# EUROPEAN PATENT SPECIFICATION

(11) **EP 4 108 297 B1**
(45) Date of publication and mention of the grant of the patent: **17.09.2025**
(21) Application number: 22180328.1
(22) Date of filing: 22.06.2022
(51) Int. Cl.: A61P 31/02, A01N 33/12, A01P 1/00, A61K 31/695, A01N 55/00, C07F 7/18

(54) **AQUEOUS SOLUTION COMPOSITION CONTAINING ORGANOSILICON COMPOUND**
WÄSSRIGE LÖSUNGSZUSAMMENSETZUNG MIT ORGANOSILIZIUMVERBINDUNG
COMPOSITION DE SOLUTION AQUEUSE CONTENANT UN COMPOSÉ D'ORGANOSILICONE

(30) Priority: 25.06.2021 JP 2021105408; 10.06.2022 JP 2022094102
(43) Date of publication of application: 28.12.2022
(73) Proprietor: Shin-Etsu Chemical Co., Ltd., Tokyo 100-0005 (JP)
(72) Inventor: HIROKAMI, Munenao, Annaka-shi (JP)
(74) Representative: Mewburn Ellis LLP

(56) References cited:
- EP-A1- 2 377 400
- GB-A- 2 407 581
- US-A1- 2013 017 242
- DATABASE WPI Week 201565, Derwent World Patents Index; AN 2015-58051R, XP002807997
- DATABASE WPI Week 2022007, Derwent World Patents Index; AN 2022-A36804, XP002807998
- DATABASE WPI Week 2022004, Derwent World Patents Index; AN 2022-554301, XP002807999

## Description

### TECHNICAL FIELD

The present invention relates to an aqueous solution composition containing a hydrolysate of a specific organosilicon compound.

### BACKGROUND

In recent years, antiviral trend has increased, and a long-acting antiviral agent is desired. For example, a composition containing octadecyldimethyl(3-trialkoxysilylpropyl)ammonium chloride as a main component is known as an immobilizable antiviral agent (Patent Documents 1 to 3).

An aqueous solution composition obtained by hydrolyzing octadecyldimethyl(3-trialkoxysilylpropyl)ammonium chloride and removing the produced alcohol is commercially available and used. But there is a disadvantage that when trying to obtain a high-concentration aqueous solution composition, it tends to gelate. There is also a problem that even a low-concentration aqueous solution composition may generate a precipitate over time. Therefore, a highly stable aqueous solution composition of a quaternary ammonium salt functional group-containing organosilicon compound has been desired.

Patent Document 4 discloses an antibacterial agent composition comprising a silicon-containing compound represented by the general formula (1) and obtained by reacting a specific triethoxysilyl compound in an ethanol solvent, and water,

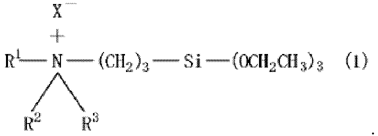

### Citation List

Patent Document 1: JP-A 2011-98976
Patent Document 2: WO 2015/141516
Patent Document 3: GB 2407581 A
Patent Document 4: EP 2377400 A1

### SUMMARY OF THE INVENTION

An object of the present invention is to provide an aqueous solution composition containing a quaternary ammonium salt functional group-containing organosilicon compound and having high storage stability.

As a result of intensive studies to achieve the above-mentioned object, the inventor has found that an aqueous solution composition containing a hydrolysate of a specific organosilicon compound is excellent in storage stability, and has completed the present invention.

In one aspect, the present invention provides an aqueous solution composition containing a hydrolysate of an organosilicon compound of formula (1): 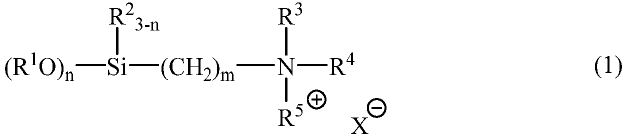 wherein R¹ and R² are each independently an alkyl group having 1 to 10 carbon atoms or an aryl group having 6 to 10 carbon atoms, R³ is an alkyl group having 12 to 24 carbon atoms, R⁴ and R⁵ are each independently an alkyl group having 1 to 6 carbon atoms, X is a halogen atom, m is an integer of 6 to 12, and n is an integer of 1 to 3.

The aqueous solution composition preferably has an alcohol content of 0.3% by weight or less with respect to the entire aqueous solution composition.

In another aspect, the invention provides a process for producing a treated article comprising the steps of treating an article with the aqueous solution composition and drying the article.

Methods of treating articles, such as fibers, with an aqueous solution according to the present invention to impart antiviral properties to the article also form part of the present disclosure.

### ADVANTAGEOUS EFFECTS

The aqueous solution composition of the present invention is excellent in storage stability, and can inhibit gelation and generation of a precipitate. Moreover, treating an article such as fibers using the aqueous solution composition of the present invention can impart antiviral properties to the article.

### FURTHER EXPLANATIONS; OPTIONS AND PREFERENCES

Hereinafter, the present invention is specifically described.

The aqueous solution composition of the present invention contains a hydrolysate of an organosilicon compound of formula (1).

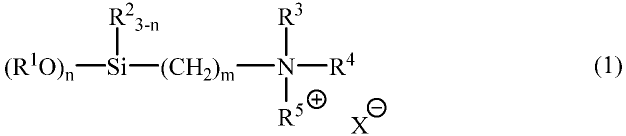

The alkyl group having 1 to 10 carbon atoms, preferably 1 to 8 carbon atoms, more preferably 1 to 6 carbon atoms as R¹ may be linear, branched, or cyclic, and specific examples thereof include methyl, ethyl, n-propyl, isopropyl, n-butyl, isobutyl, s-butyl, t-butyl, n-pentyl, n-hexyl, and cyclohexyl groups.

Specific examples of the aryl group having 6 to 10 carbon atoms, preferably 6 to 8 carbon atoms include a phenyl and a tolyl groups.

Among them, an alkyl group having 1 to 3 carbon atoms is preferred, and a methyl group or an ethyl group is more preferred for R¹.

Examples of the alkyl group having 1 to 10 carbon atoms and the aryl group having 6 to 10 carbon atoms as R² include groups same as those mentioned for R¹, and among them, a methyl group is more preferred.

The alkyl group having 12 to 24 carbon atoms, preferably 12 to 20 carbon atoms, more preferably 12 to 18 carbon atoms as R³ may be linear, branched, or cyclic, and specific examples thereof include n-dodecyl, 2-methylundecyl, n-tridecyl, n-tetradecyl, n-pentadecyl, n-hexadecyl, n-heptadecyl, n-octadecyl, n-nonadecyl, n-eicosyl, n-heneicosyl, n-docosyl, n-tricosyl, n-tetracosyl, and cyclododecyl groups. Among them, an alkyl group having 14 to 18 carbon atoms is preferred for R³, and a n-octadecyl group is more preferred for R³ from the viewpoint of availability of raw materials and environmental load during use.

The alkyl group having 1 to 6 carbon atoms as R⁴ and R⁵ may be linear, branched, or cyclic, and specific examples thereof include methyl, ethyl, n-propyl, isopropyl, n-butyl, isobutyl, s-butyl, t-butyl, n-pentyl, n-hexyl, and cyclohexyl groups. Among them, an alkyl group having 1 to 3 carbon atoms is preferred for both R⁴ and R⁵, and a methyl group is more preferred for R⁴ and R⁵ from the viewpoint of availability of raw materials and environmental load during use.

m represents an integer of 6 to 12, preferably 8. If m is less than 4, the aqueous solution composition is insufficient in storage stability, whereas if m is more than 20, the quaternary ammonium salt content per unit mass is reduced, so that the antiviral properties are reduced.

Examples of the halogen atom as X include a chlorine atom and a bromine atom.

Specific examples of the organosilicon compound usable in the aqueous solution composition of the present invention include
octadecyldimethyl(6-trimethoxysilylhexyl)ammonium chloride,
octadecyldimethyl(6-triethoxysilylhexyl)ammonium chloride,
octadecyldimethyl(8-trimethoxysilyloctyl)ammonium chloride,
octadecyldimethyl(8-triethoxysilyloctyl)ammonium chloride,
octadecyldimethyl(8-dimethoxymethylsilyloctyl)ammonium chloride,
octadecyldimethyl(8-diethoxymethylsilyloctyl)ammonium chloride,
octadecyldimethyl(10-trimethoxysilyldecyl)ammonium chloride,
octadecyldimethyl(10-triethoxysilyldecyl)ammonium chloride,
octadecyldimethyl(11-trimethoxysilylundecyl)ammonium chloride,
octadecyldimethyl(11-triethoxysilylundecyl)ammonium chloride,
octadecyldimethyl(12-trimethoxysilyldodecyl)ammonium chloride,
octadecyldimethyl(12-triethoxysilyldodecyl)ammonium chloride,
dodecyldimethyl(8-triethoxysilyloctyl)ammonium chloride,
dodecyldimethyl(8-dimethoxymethylsilyloctyl)ammonium chloride,
tetradecyldimethyl(8-triethoxysilyloctyl)ammonium chloride,
tetradecyldimethyl(8-dimethoxymethylsilyloctyl)ammonium chloride,
octadecyldiethyl(8-triethoxysilyloctyl)ammonium chloride, and
octadecyldiethyl(8-dimethoxymethylsilyloctyl)ammonium chloride. These may be used alone or in combination of two or more kinds thereof.

Among them, octadecyldimethyl(6-triethoxysilylhexyl)ammonium chloride, octadecyldimethyl(8-trimethoxysilyloctyl)ammonium chloride, octadecyldimethyl(8-triethoxysilyloctyl)ammonium chloride, octadecyldimethyl(8-dimethoxymethylsilyloctyl)ammonium chloride, octadecyldimethyl(8-diethoxymethylsilyloctyl)ammonium chloride, and octadecyldimethyl(11-trimethoxysilylundecyl)ammonium chloride are preferred, and octadecyldimethyl(8-trimethoxysilyloctyl)ammonium chloride, octadecyldimethyl(8-triethoxysilyloctyl)ammonium chloride, octadecyldimethyl(8-dimethoxymethylsilyloctyl)ammonium chloride, and octadecyldimethyl(8-diethoxymethylsilyloctyl)ammonium chloride are more preferred.

The organosilicon compound of formula (1) is obtained by reacting an organosilicon compound of formula (A) with a tertiary amine of formula (B) in an air atmosphere or an atmosphere of an inert gas such as nitrogen.

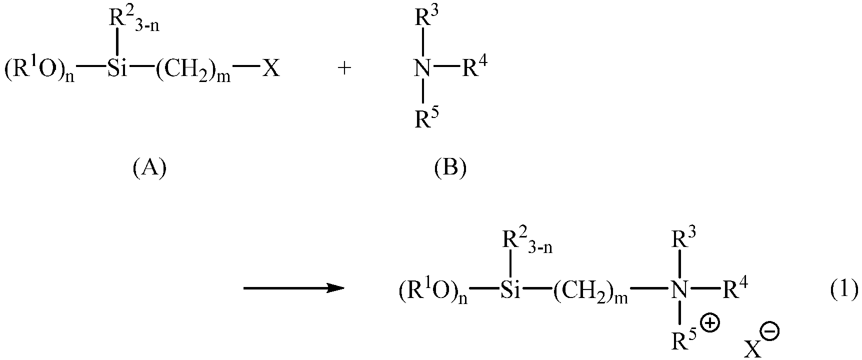

In the formulae, R¹, R², R³, R⁴, R⁵, X, m, and n are as defined above.

Examples of the compound of formula (A) include
6-chlorohexyltrimethoxysilane, 6-chlorohexyltriethoxysilane,
6-bromohexyltrimethoxysilane, 6-bromohexyltriethoxysilane,
8-chlorooctyltrimethoxysilane, 8-chlorooctyltriethoxysilane,
8-chlorooctyldimethoxymethylsilane, 8-chlorooctyldiethoxymethylsilane,
8-bromooctyltrimethoxysilane, 8-bromooctyltriethoxysilane,
8-bromooctyldimethoxymethylsilane, 8-bromooctyldiethoxymethylsilane,
10-chlorodecyltrimethoxysilane, 10-chlorodecyltriethoxysilane,
10-bromodecyltrimethoxysilane, 10-bromodecyltriethoxysilane,
11-chloroundecyltrimethoxysilane, 11-chloroundecyltriethoxysilane,
11-bromoundecyltrimethoxysilane, 11-bromoundecyltriethoxysilane,
12-chlorododecyltrimethoxysilane, 12-chlorododecyltriethoxysilane,
12-bromododecyltrimethoxysilane, and 12-bromododecyltriethoxysilane. These can be used alone or in combination of two or more kinds thereof.

Among them, 6-chlorohexyltrimethoxysilane, 6-chlorohexyltriethoxysilane, 8-chlorooctyltrimethoxysilane, 8-chlorooctyltriethoxysilane, 8-chlorooctyldimethoxymethylsilane, 8-chlorooctyldiethoxymethylsilane, 11-chloroundecyltrimethoxysilane, and 11-chloroundecyltriethoxysilane are preferred, and 8-chlorooctyltrimethoxysilane, 8-chlorooctyltriethoxysilane, 8-chlorooctyldimethoxymethylsilane, and 8-chlorooctyldiethoxymethylsilane are more preferred.

Examples of the tertiary amine of formula (B) include dodecyldimethylamine, dodecyldiethylamine, tridecyldimethylamine, tridecyldiethylamine, tetradecyldimethylamine, tetradecyldiethylamine, pentadecyldimethylamine, pentadecyldiethylamine, hexadecyldimethylamine, hexadecyldiethylamine, heptadecyldimethylamine, heptadecyldiethylamine, octadecyldimethylamine, octadecyldiethylamine, nonadecyldimethylamine, nonadecyldiethylamine, eicosyldimethylamine, eicosyldiethylamine, heneicosyldimethylamine, heneicosyldiethylamine, docosyldimethylamine, docosyldiethylamine, tricosyldimethylamine, tricosyldiethylamine, tetracosyldimethylamine, and tetracosyldiethylamine. These can be used alone or in combination of two or more kinds thereof.

Among them, octadecyldimethylamine and octadecyldiethylamine are preferred, and octadecyldimethylamine is more preferred.

The above-mentioned reaction can be carried out in the absence of a solvent, but can also be carried out in an alcohol solvent such as methanol or ethanol if necessary as long as the reaction is not inhibited.

The reaction temperature is preferably 80 to 150°C, and more preferably 100 to 130°C. The reaction time is preferably 1 to 30 hours, and more preferably 5 to 25 hours.

In the reaction, the use ratio of the organosilicon compound of formula (A) to the tertiary amine of formula (B) is preferably 0.7 to 1.3 mol of the organosilicon compound (A) to 1 mol of the tertiary amine (B).

The aqueous solution composition of the present invention is obtained by diluting the organosilicon compound of formula (1) with water in an air atmosphere or an atmosphere of an inert gas such as nitrogen, and hydrolyzing a part or all of groups represented by Si-OR¹ (R¹ is the same as described above) in formula (1) to silanol groups (Si-OH groups). In addition, water may be added continuously during the hydrolysis reaction, or may be added before starting the reaction. An alcohol solution of the organosilicon compound of formula (1) obtained by the above-mentioned reaction can be used as it is in the hydrolysis reaction.

The temperature of the hydrolysis reaction is preferably 50 to 110°C, and more preferably 60 to 105°C. The reaction time is preferably 1 to 30 hours, and more preferably 5 to 25 hours.

An alcohol component (R¹OH (R¹ is the same as described above)) by-produced by the hydrolysis reaction and an alcohol component added together with water as needed in the hydrolysis reaction are preferably removed by a separation method such as distillation. The distillation is preferably performed simultaneously with the hydrolysis reaction.

The amount of the hydrolysate of the organosilicon compound of formula (1) contained in the aqueous solution composition of the present invention is not particularly limited, but is preferably 0.1 to 50% by weight, more preferably 0.5 to 20% by weight, still more preferably more than 0.5% by weight and 15% by weight or less, and particularly preferably 1 to 10% by weight with respect to an entirety of the aqueous solution composition from the viewpoint of storage stability and productivity.

The amount of alcohol contained in the aqueous solution composition of the present invention is preferably 0.3% by weight or less, and more preferably 0.1% by weight or less with respect to an entirety of the aqueous solution composition from the viewpoint of safety. The amount of alcohol in the aqueous solution composition can be determined by gas chromatography (GC) analysis.

The aqueous solution composition of the present invention may contain an organic acid such as citric acid, and an additive such as a surfactant, as long as the object of the present invention is not impaired, and the aqueous solution composition preferably contains an organic acid such as citric acid from the viewpoint of storage stability of the aqueous solution.

The aqueous solution composition of the present invention can be applied to various materials and articles to impart antibacterial properties and antiviral properties to such materials and articles. Specific examples of the materials and articles include various fiber materials including natural fibers such as cotton, silk, and wool, regenerated fibers such as rayon, semi-synthetic fibers such as acetate, synthetic fibers such as vinylon, polyester, nylon, polyethylene, polypropylene, polyurethane, and polyaramid fibers, and composite fibers of these fibers (such as polyester/cotton); various metal materials including iron, stainless steel, aluminum, nickel, zinc, and copper; various synthetic resin materials including an acrylic resin, a phenol resin, an epoxy resin, a polycarbonate resin, and a polybutylene terephthalate resin; various inorganic materials including glass, titanium, ceramic, cement, and mortar; and various articles made from these materials.

### EXAMPLES

Hereinafter, the present invention is described in more detail with reference to Synthesis Examples, a Comparative Synthesis Example, Examples, and Comparative Examples.

### Synthesis of organosilicon compound

### Reference Synthesis Example 1-1

In a 300-mL pressurized reaction vessel purged with nitrogen, 42.4 g of 4-chlorobutyltrimethoxysilane, 59.6 g of octadecyldimethylamine (LIPOMIN DM18D, manufactured by Lion Corporation, the same applies hereinafter), and 102 g of methanol were charged, and reacted at 120°C for 20 hours. After the reaction, filtration was performed to obtain 200 g of a methanol solution of octadecyldimethyl(4-trimethoxysilylbutyl)ammonium chloride (solid content concentration: 50% by weight).

### Synthesis Example 1-2

In a 300-mL pressurized reaction vessel purged with nitrogen, 48.0 g of 6-chlorohexyltrimethoxysilane, 59.6 g of octadecyldimethylamine, and 108 g of methanol were charged, and reacted at 120°C for 20 hours. After the reaction, filtration was performed to obtain 206 g of a methanol solution of octadecyldimethyl(6-trimethoxysilylhexyl)ammonium chloride (solid content concentration: 50% by weight).

### Synthesis Example 1-3

In a 300-mL pressurized reaction vessel purged with nitrogen, 53.8 g of 8-chlorooctyltrimethoxysilane, 59.6 g of octadecyldimethylamine, and 113 g of methanol were charged, and reacted at 120°C for 20 hours. After the reaction, filtration was performed to obtain 220 g of a methanol solution of octadecyldimethyl(8-trimethoxysilyloctyl)ammonium chloride (solid content concentration: 50% by weight).

### Synthesis Example 1-4

In a 300-mL pressurized reaction vessel purged with nitrogen, 62.2 g of 8-chlorooctyltriethoxysilane, 59.6 g of octadecyldimethylamine, and 122 g of ethanol were charged, and reacted at 120°C for 20 hours. After the reaction, filtration was performed to obtain 235 g of an ethanol solution of octadecyldimethyl(8-triethoxysilyloctyl)ammonium chloride (solid content concentration: 50% by weight).

### Synthesis Example 1-5

In a 300-mL pressurized reaction vessel purged with nitrogen, 62.2 g of 11-chloroundecyltrimethoxysilane, 59.6 g of octadecyldimethylamine, and 122 g of methanol were charged, and reacted at 120°C for 20 hours. After the reaction, filtration was performed to obtain 235 g of a methanol solution of octadecyldimethyl(11-trimethoxysilylundecyl)ammonium chloride (solid content concentration: 50% by weight).

### Comparative Synthesis Example 1-1

In a 300-mL pressurized reaction vessel purged with nitrogen, 39.7 g of 3-chloropropyltrimethoxysilane, 59.6 g of octadecyldimethylamine, and 99.3 g of methanol were charged, and reacted at 120°C for 20 hours. After the reaction, filtration was performed to obtain 190 g of a methanol solution of octadecyldimethyl(3-trimethoxysilylpropyl)ammonium chloride (solid content concentration: 50% by weight).

### Production of aqueous solution composition

### Reference Example 1-1

In a 500-mL pressurized reaction vessel purged with nitrogen, 10 g of the methanol solution of octadecyldimethyl(4-trimethoxysilylbutyl)ammonium chloride (solid content concentration: 50% by weight) obtained in Reference Synthesis Example 1-1, 200 g of ion-exchanged water, and 0.4 g of citric acid were charged. The water-methanol mixed solvent was distilled off until the internal temperature reached 100°C, and then ion-exchanged water was added so that the concentration became 3% by weight, thereby obtaining an aqueous solution composition A.

The obtained aqueous solution composition had a transparent appearance, and methanol was not detected in the GC analysis.

### Example 1-2

In a 500-mL pressurized reaction vessel purged with nitrogen, 10 g of the methanol solution of octadecyldimethyl(6-trimethoxysilylhexyl)ammonium chloride (solid content concentration: 50% by weight) obtained in Synthesis Example 1-2, 200 g of ion-exchanged water, and 0.4 g of citric acid were charged. The water-methanol mixed solvent was distilled off until the internal temperature reached 100°C, and then ion-exchanged water was added so that the concentration became 3% by weight, thereby obtaining an aqueous solution composition B.

The obtained aqueous solution composition had a transparent appearance, and methanol was not detected in the GC analysis.

### Example 1-3

In a 500-mL pressurized reaction vessel purged with nitrogen, 10 g of the methanol solution of octadecyldimethyl(8-trimethoxysilyloctyl)ammonium chloride (solid content concentration: 50% by weight) obtained in Synthesis Example 1-3, 200 g of ion-exchanged water, and 0.4 g of citric acid were charged. The water-methanol mixed solvent was distilled off until the internal temperature reached 100°C, and then ion-exchanged water was added so that the concentration became 3% by weight, thereby obtaining an aqueous solution composition C.

The obtained aqueous solution composition had a transparent appearance, and methanol was not detected in the GC analysis.

### Example 1-4

In a 500-mL pressurized reaction vessel purged with nitrogen, 10 g of the ethanol solution of octadecyldimethyl(8-triethoxysilyloctyl)ammonium chloride (solid content concentration: 50% by weight) obtained in Synthesis Example 1-4, 200 g of ion-exchanged water, and 0.4 g of citric acid were charged. The water-ethanol mixed solvent was distilled off until the internal temperature reached 100°C, and then ion-exchanged water was added so that the concentration became 3% by weight, thereby obtaining an aqueous solution composition D.

The obtained aqueous solution composition had a transparent appearance, and ethanol was not detected in the GC analysis.

### Example 1-5

In a 500-mL pressurized reaction vessel purged with nitrogen, 10 g of the ethanol solution of octadecyldimethyl(8-triethoxysilyloctyl)ammonium chloride (solid content concentration: 50% by weight) obtained in Synthesis Example 1-4, 150 g of ion-exchanged water, and 0.4 g of citric acid were charged. The water-ethanol mixed solvent was distilled off until the internal temperature reached 100°C, and then ion-exchanged water was added so that the concentration became 5% by weight, thereby obtaining an aqueous solution composition E.

The obtained aqueous solution composition had a transparent appearance, and ethanol was not detected in the GC analysis.

### Example 1-6

In a 500-mL pressurized reaction vessel purged with nitrogen, 10 g of the ethanol solution of octadecyldimethyl(8-triethoxysilyloctyl)ammonium chloride (solid content concentration: 50% by weight) obtained in Synthesis Example 1-4, 400 g of ion-exchanged water, and 0.4 g of citric acid were charged. The water-ethanol mixed solvent was distilled off until the internal temperature reached 100°C, and then ion-exchanged water was added so that the concentration became 1% by weight, thereby obtaining an aqueous solution composition F.

The obtained aqueous solution composition had a transparent appearance, and ethanol was not detected in the GC analysis.

### Example 1-7

In a 500-mL pressurized reaction vessel purged with nitrogen, 10 g of the methanol solution of octadecyldimethyl(11-trimethoxysilylundecyl)ammonium chloride (solid content concentration: 50% by weight) obtained in Synthesis Example 1-5, 200 g of ion-exchanged water, and 0.4 g of citric acid were charged. The water-methanol mixed solvent was distilled off until the internal temperature reached 100°C, and then ion-exchanged water was added so that the concentration became 3% by weight, thereby obtaining an aqueous solution composition G.

The obtained aqueous solution composition had a transparent appearance, and methanol was not detected in the GC analysis.

### Comparative Example 1-1

In a 500-mL pressurized reaction vessel purged with nitrogen, 10 g of the methanol solution of octadecyldimethyl(3-trimethoxysilylpropyl)ammonium chloride (solid content concentration: 50% by weight) obtained in Comparative Synthesis Example 1-1, 150 g of ion-exchanged water, and 0.4 g of citric acid were charged. The water-methanol mixed solvent was distilled off until the internal temperature reached 100°C, and then ion-exchanged water was added so that the concentration became 5% by weight, thereby obtaining an aqueous solution composition H.

The obtained aqueous solution composition had a transparent appearance, and methanol was not detected in the GC analysis.

### Comparative Example 1-2

In a 500-mL pressurized reaction vessel purged with nitrogen, 10 g of the methanol solution of octadecyldimethyl(3-trimethoxysilylpropyl)ammonium chloride (solid content concentration: 50% by weight) obtained in Comparative Synthesis Example 1-1, 200 g of ion-exchanged water, and 0.4 g of citric acid were charged. The water-methanol mixed solvent was distilled off until the internal temperature reached 100°C, and then ion-exchanged water was added so that the concentration became 3% by weight, thereby obtaining an aqueous solution composition I.

The obtained aqueous solution composition had a transparent appearance, and methanol was not detected in the GC analysis.

### Comparative Example 1-3

In a 500-mL pressurized reaction vessel purged with nitrogen, 10 g of the methanol solution of octadecyldimethyl(3-trimethoxysilylpropyl)ammonium chloride (solid content concentration: 50% by weight) obtained in Comparative Synthesis Example 1-1, 400 g of ion-exchanged water, and 0.4 g of citric acid were charged. The water-methanol mixed solvent was distilled off until the internal temperature reached 100°C, and then ion-exchanged water was added so that the concentration became 1% by weight, thereby obtaining an aqueous solution composition J.

The obtained aqueous solution composition had a transparent appearance, and methanol was not detected in the GC analysis.

### Evaluation of storage stability

The aqueous solution compositions A to J obtained in Reference Example 1-1, Examples 1-2 to 1-7 and Comparative Examples 1-1 to 1-3 were stored under sealed conditions at 50°C for a predetermined period (1 week, 1 month, and 3 months), and then the appearance of the compositions was visually observed to evaluate the stability. A case where no precipitate was observed and the solution was transparent was evaluated as good, and a case where a precipitate was observed was evaluated as poor. The results are shown in Table 1.

**Table 1**

| | Aqueous solution composition | Stability (storage at 50°C) | | |
|---|---|---|---|---|
| | | 1 week | 1 month | 3 months |
| Reference Example 1-1 | A | good | good | poor |
| Example 1-2 | B | good | good | good |
| Example 1-3 | C | good | good | good |
| Example 1-4 | D | good | good | good |
| Example 1-5 | E | good | good | good |
| Example 1-6 | F | good | good | good |
| Example 1-7 | G | good | good | good |
| Comparative Example 1-1 | H | poor | poor | poor |
| Comparative Example 1-2 | I | good | poor | poor |
| Comparative Example 1-3 | J | good | good | poor |

### Production of treated article

### Reference Example 2-1, Examples 2-2 to 2-7 and Comparative Examples 2-1 to 2-3

Each of the aqueous solution compositions A to J obtained in Reference Example 1-1, Examples 1-2 to 1-7 and Comparative Examples 1-1 to 1-3 was further diluted with ion-exchanged water to a concentration of 0.5% by weight, 5 g of T/C fibers (polyester/cotton composite fibers) were immersed in each of the aqueous solution compositions A to J for 30 seconds, and the T/C fibers were taken out and dried at 80°C for 10 minutes to produce treated T/C fibers.

### Evaluation of antiviral properties

The obtained fibers were evaluated for antiviral properties by the following method. The evaluation was performed according to the virus reduction rate calculated by the following method. The results are shown in Table 2.

| | |
|---|---|
| Fibers: | T/C fibers that were treated with an antiviral agent composition and washed 5 times and 10 times in a home washing machine (model No. NA-VX86002, manufactured by Panasonic Corporation, standard course, detergent: JAFET standard detergent) |
| Virus used: | Influenza virus H1N1 IOWA strain |
| Culture cells: | MDCK cells |

Method for preparing virus fluid:
The influenza virus was inoculated into the MDCK cells. After adsorption at 37°C for 1 hour, a fluid of the inoculated virus was removed, and washed twice with sterile PBS. A MEM medium was added to the fluid, and the fluid was cultured at 37°C in the presence of 5% CO₂ for 5 days. The culture supernatant was collected and centrifuged at 3000 rpm for 30 minutes, and then the centrifuged supernatant was dispensed and stored at -70°C or lower to obtain a virus fluid.

Measurement of virus titer:
To the above-mentioned fibers, 0.4 mL of the virus fluid was added, and the fibers were sealed in a sterile vial. The fibers were allowed to stand at room temperature (25°C) for a sensitization time of 2 hours. After the lapse of the sensitization time, 20 mL of a cell maintenance medium was added to the vial and mixed well to wash out the virus. The washout was further subjected to 10-fold serial dilution with a cell maintenance medium, and each of the diluted solutions was inoculated into the culture cells in a microplate and cultured at 37°C for 5 days. Each of the culture solutions after the culture was collected, and whether the virus was proliferated was determined by a hemagglutination reaction to measure the virus titer (TCID50). 100 × [(virus titer in untreated fibers - virus titer in the above-mentioned fibers) / (virus titer in untreated fibers)]

**Table 2**

| | Aqueous solution composition | Virus reduction rate (%) | | |
|---|---|---|---|---|
| | | 0 times of washing | 5 times of washing | 10 times of washing |
| Reference Example 2-1 | A | 99.9 or more | 99.0 | 50 |
| Example 2-2 | B | 99.9 or more | 99.9 or more | 99.0 |
| Example 2-3 | C | 99.9 or more | 99.9 or more | 99.9 or more |
| Example 2-4 | D | 99.9 or more | 99.9 or more | 99.9 or more |
| Example 2-5 | E | 99.9 or more | 99.9 or more | 99.9 or more |
| Example 2-6 | F | 99.9 or more | 99.9 or more | 99.9 or more |
| Example 2-7 | G | 99.9 or more | 99.9 or more | 99.0 |
| Comparative Example 2-1 | H | 99.9 or more | 80 | 0 |
| Comparative Example 2-2 | I | 99.9 or more | 80 | 0 |
| Comparative Example 2-3 | J | 99.9 or more | 80 | 0 |

As shown in Tables 1 and 2, the aqueous solution compositions of Reference Example 1-1, Examples 1-2 to 1-7 were superior in storage stability to the aqueous solution compositions of Comparative Examples 1-1 to 1-3, and were also superior in washing durability of antiviral properties of the treated fibers to the aqueous solution compositions of Comparative Examples 1-1 to 1-3.

## Claims

1. An aqueous solution composition comprising a hydrolysate of an organosilicon compound of formula (1): 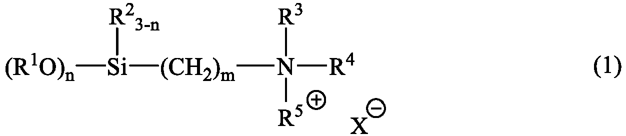 wherein R¹ and R² are each independently an alkyl group having 1 to 10 carbon atoms or an aryl group having 6 to 10 carbon atoms, R³ is an alkyl group having 12 to 24 carbon atoms, R⁴ and R⁵ are each independently an alkyl group having 1 to 6 carbon atoms, X is a halogen atom, m is an integer of 6 to 12, and n is an integer of 1 to 3.

2. The aqueous solution composition according to claim 1, wherein the alcohol content is 0.3% by weight or less with respect to the entire aqueous solution composition.

3. The aqueous solution composition according to claim 1 or 2, wherein the amount of the hydrolysate of the organosilicon compound of formula (1) in the composition is 0.1 to 50% by weight.

4. The aqueous solution composition according to any of claims 1 to 3, wherein R¹ is each independently a methyl group or an ethyl group, and R² is a methyl group.

5. The aqueous solution composition according to any of claims 1 to 4, wherein the organosilicon compound of formula (1) is selected from octadecyldimethyl(6-triethoxysilylhexyl)ammonium chloride, octadecyldimethyl(8-trimethoxysilyloctyl)ammonium chloride, octadecyldimethyl(8-triethoxysilyloctyl)ammonium chloride, octadecyldimethyl(8-dimethoxymethylsilyloctyl)ammonium chloride, octadecyldimethyl(8-diethoxymethylsilyloctyl)ammonium chloride, and octadecyldimethyl(11-trimethoxysilylundecyl)ammonium chloride.

6. A process for producing a treated article comprising the steps of treating an article with the aqueous solution composition according to any one of claims 1 to 5 and drying the article.

7. Use of the aqueous solution composition according to any one of claims 1 to 5 for imparting antiviral properties to an article.

## Patentansprüche

1. Wässrige Lösungszusammensetzung, die ein Hydrolysat einer Organosilicium-Verbindung der Formel (1) umfasst: 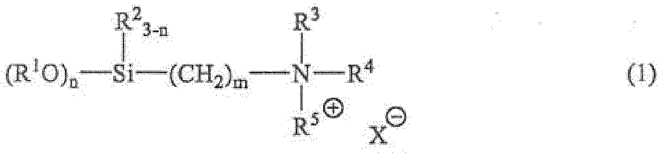 worin R¹ und R² jeweils unabhängig voneinander eine Alkylgruppe mit 1 bis 10 Kohlenstoffatomen oder eine Arylgruppe mit 6 bis 10 Kohlenstoffatomen sind, R³ eine Alkylgruppe mit 12 bis 24 Kohlenstoffatomen ist, R⁴ und R⁵ jeweils unabhängig voneinander eine Alkylgruppe mit 1 bis 6 Kohlenstoffatomen sind, X ein Halogenatom ist, m eine ganze Zahl von 6 bis 12 ist und n eine ganze Zahl von 1 bis 3 ist.

2. Wässrige Lösungszusammensetzung nach Anspruch 1, wobei der Alkoholgehalt 0,3 Gew.-% oder weniger, bezogen auf die gesamte wässrige Lösungszusammensetzung beträgt.

3. Wässrige Lösungszusammensetzung nach Anspruch 1 oder 2, wobei die Menge des Hydrolysats der Organosilicium-Verbindung der Formel (1) in der Zusammensetzung 0,1 bis 50 Gew.-% beträgt.

4. Wässrige Lösungszusammensetzung nach einem der Ansprüche 1 bis 3, wobei R¹ jeweils unabhängig eine Methylgruppe oder eine Ethylgruppe ist und R² eine Methylgruppe ist.

5. Wässrige Lösungszusammensetzung nach einem der Ansprüche 1 bis 4, wobei die Organosilicium-Verbindung der Formel (1) aus Octadecyldimethyl(6-triethoxysilylhexyl)ammoniumchlorid, Octadecyldimethyl(8-trimethoxysilyloctyl)ammoniumchlorid, Octadecyldimethyl(8-triethoxysilyloctyl)ammoniumchlorid, Octadecyldimethyl(8-dimethoxymethylsilyloctyl)ammoniumchlorid, Octadecyldimethyl(8-diethoxymethylsilyloctyl)ammoniumchlorid und Octadecyldimethyl(11-trimethoxysilylundecyl)ammoniumchlorid ausgewählt ist.

6. Verfahren zur Herstellung eines behandelten Erzeugnisses, das die Schritte des Behandelns eines Erzeugnisses mit einer wässrigen Lösungszusammensetzung nach einem der Ansprüche 1 bis 5 und des Trocknens des Erzeugnisses umfasst.

7. Verwendung einer wässrigen Lösungszusammensetzung nach einem der Ansprüche 1 bis 5, um einem Erzeugnis antivirale Eigenschaften zu verleihen.

## Revendications

1. Composition de solution aqueuse comprenant un hydrolysat d'un composé organosilicié de formule (1) : 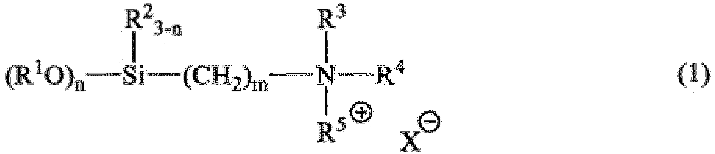 dans laquelle R¹ et R² sont chacun indépendamment un groupe alkyle présentant de 1 à 10 atomes de carbone ou un groupe aryle présentant de 6 à 10 atomes de carbone, R³ est un groupe alkyle présentant de 12 à 24 atomes de carbone, R⁴ et R⁵ sont chacun indépendamment un groupe alkyle présentant de 1 à 6 atomes de carbone, X est un atome d'halogène, m est un nombre entier de 6 à 12, et n est un nombre entier de 1 à 3.

2. Composition de solution aqueuse selon la revendication 1, dans laquelle la teneur en alcool est de 0,3 % en poids ou moins par rapport à la totalité de la composition de solution aqueuse.

3. Composition de solution aqueuse selon la revendication 1 ou 2, dans laquelle la quantité de l'hydrolysat du composé organosilicié de formule (1) dans la composition est de 0,1 à 50 % en poids.

4. Composition de solution aqueuse selon l'une quelconque des revendications 1 à **3,** dans laquelle chaque R¹ est indépendamment un groupe méthyle ou un groupe éthyle, et R² est un groupe méthyle.

5. Composition de solution aqueuse selon l'une quelconque des revendications 1 à 4, dans laquelle le composé organosilicié de formule (1) est choisi parmi le chlorure d'octadécyldiméthyl(6-triéthoxysilylhexyl)ammonium, le chlorure d'octadécyldiméthyl(8-triméthoxy-silyloctyl)ammonium, le chlorure d'octadécyldiméthyl(8-triéthoxysilyloctyl) ammonium, le chlorure d'octadécyl-diméthyl(8-diméthoxyméthylsilyloctyl)ammonium, le chlorure d'octadécyldiméthyl(8-diéthoxyméthylsilyloctyl)ammonium et le chlorure d'octadécyldiméthyl(11-triméthoxy-silylundécyl) ammonium.

6. Procédé de production d'un article traité, comprenant les étapes consistant à traiter un article avec la composition de solution aqueuse selon l'une quelconque des revendications 1 à 5 et à sécher l'article.

7. Utilisation de la composition de solution aqueuse selon l'une quelconque des revendications 1 à 5 pour conférer des propriétés antivirales à un article.
